# EUROPEAN PATENT APPLICATION

(11) **EP 3 923 298 A1**
(43) Date of publication of application: **15.12.2021**
(21) Application number: 20179446.8
(22) Date of filing: 11.06.2020
(51) Int. Cl.: G16H 40/63, G16H 40/40

(54) **IMAGE-BASED ADAPTING OF MEDICAL EQUIPMENT**

(71) Applicant: Koninklijke Philips N.V., 5656 AG Eindhoven (NL)
(72) Inventor: RONGEN, Peter Maria Johannes, 5656 AE Eindhoven (NL); POST, Wijnand, 5656 AE Eindhoven (NL); VAN RIEL, Sjors, 5656 AE Eindhoven (NL); OLIVAN BESCOS, Javier, 5656 AE Eindhoven (NL); DE PAUW, Robert-Jan, 5656 AE Eindhoven (NL)
(74) Representative: Philips Intellectual Property & Standards

(57) **Abstract**

In order to provide further relief for staff members, a device (10) for procedure-based adaptation of medical equipment comprises an image data receiver (12) as an input, a processor (14) and an equipment adaptor (16) as an output. Images of a situation that show activity in a medical procedure zone are used to detect individuals and medical equipment in the medical procedure zone and their corresponding activities, and to recognize activity patterns and roles based on the detected activities. Adaptation possibilities of the medical equipment are analyzed and adjustment values for the medical equipment is determined. The equipment adaptor provides adapted control signals for the medical equipment based on the determined adjustment values. In an example, in a cath lab (200), a system (100) comprises an image capturing arrangement (102) with cameras that provide the images. The adapted control signals are used for adapting medical equipment (112) of the cath lab.

## Description

### FIELD OF THE INVENTION

The present invention relates to adapting medical equipment, and relates in particular to a device for procedure-based adaptation of medical equipment, to a system for procedure-based adaptation of medical equipment in an interventional space and to a method for procedure-based adaptation of medical equipment.

### BACKGROUND OF THE INVENTION

During medical procedures such as examinations or interventions, different type of medical equipment is used. As an example, during minimal invasive cath lab interventions for treating patients suffering from, for instance, cardiovascular diseases, clinical staff may use a large number of electronic and medical equipment like imaging devices, such as X-ray or ultrasound imaging systems etc., and disposable intervention tooling, as well as visualization tooling etc. Further, ambient environment control and the like may also be provided. It is thus possible to improve workflow etc. by assorted cath lab setups. However, it has been shown that an increase in the number of equipment also leads to more complex operating procedures of interacting medical equipment that can be cumbersome for the clinical staff to operate in an efficient manner. For staff members, the control of the equipment can also be distracting from their original tasks.

### SUMMARY OF THE INVENTION

There may thus be a need to provide further support and relief for staff members during workflows in a medical procedure.
The object of the present invention is solved by the subject-matter of the independent claims; further embodiments are incorporated in the dependent claims. It should be noted that the following described aspects of the invention apply also for the device for procedure-based adaptation of medical equipment, for the system for procedure-based adaptation of medical equipment in an interventional space and for the method for procedure-based adaptation of medical equipment.

According to the present invention, a device for procedure-based adaptation of medical equipment is provided. The device comprises an image data receiver, a processor and an equipment adaptor. The image data receiver is configured to receive image data of a situation in a medical procedure zone comprising medical equipment, which image data shows at least a part of an activity in the medical procedure zone. The processor is configured to detect at least one of the group of individuals and medical equipment in the medical procedure zone and their corresponding activities based on the image data, to recognize activity patterns and roles based on the detected activities, to analyze adaptation possibilities of the medical equipment based on the recognized activity patterns and roles, and to determine adjustment values for the medical equipment based on the analyzed adaptation possibilities. For a procedure-based adaptation of the medical equipment, the equipment adaptor is configured to provide adapted control signals for the medical equipment based on the determined adjustment values.

Thus, the equipment can be adjusted to serve in a more efficient and user-friendly manner. The adaptation provides relief for the staff members. Since the adapting of the medical equipment is image-based, additional equipment can be considered without the need to provide special treatment or manipulation of such additional equipment. The image-based adapting of medical equipment is thus also suitable for upgrading existing arrangements and systems.

According to an example, the adapted control signals comprise technically related data resulting in an adaptation of the medical equipment.

According to an example, the processor is configured to predict activities and roles to follow the detected activities based on the recognized activity patterns and roles, and to analyze adaptation possibilities of the medical equipment based on the predicted activity patterns and roles, and to determine the adjustment values based on the analyzed adaptation possibilities.

According to an example, the processor is provided with an artificial intelligence deep learning device for role or activity detection. Alternatively, or in addition, the processor is provided with an artificial neural network comprising a model embedded in the processor for processing the image data.

According to the present invention, also a system for procedure-based adaptation of medical equipment in an interventional space is provided. The system comprises an image capturing arrangement, and one of the above-described examples of the device for procedure-based adaptation of medical equipment. The image capturing arrangement provides a plurality of images as the image data to the image data receiver. The equipment adaptor is configured to provide the adapted control signals for at least a part of the medical equipment based on the image data from the image capturing arrangement.

According to an example, the system is a cathlab system comprising medical equipment. The equipment adaptor provides the adapted control signals to at least a part of the medical equipment. Further, at least a part of the medical equipment operates according the adapted control signals.

Thus, a smart dynamically adapting intervention lab is provided based on activity and role detection.

According to the present invention, also a method for procedure-based adaptation of medical equipment is provided. The method comprises the following steps:
- receiving image data of a situation in a medical procedure zone comprising medical equipment, which image data shows at least a part of an activity in the medical procedure zone;
- detecting at least one of the group of individuals and medical equipment in the medical procedure zone and their corresponding activities based on the image data;
- recognizing activity patterns and roles based on the detected activities;
- analyzing adaptation possibilities of the medical equipment based on the recognized activity patterns and roles;
- determining adjustment values for the medical equipment based on the analyzed adaptation possibilities; and
- providing adapted control signals for the medical equipment based on the determined adjustment values for a procedure-based adaptation of the medical equipment.

According to an aspect, a video grabbing system is provided that records persons, i.e. actors, in the control and intervention room of a cath lab. A data processor analyses activity patterns and detects the type of persons and their corresponding roles in the procedure. This operates with either computer vision or artificial intelligence deep learning components. A certain action is then performed, depending on the detected activity or role, to adapt the operating environment of the corresponding staff member(s). This adaptation is experienced as a so-to-speak adaptive intelligent behavior of the cath lab itself.

The artificial intelligence may comprise a dedicated trained (deep) neural network and model, embedded in a dedicated processor, e.g. inside the observation camera itself. With such a specific platform, the deep learning model and network weights are exchangeable with other models and weights, in order to easily change the adaptive intelligent behavior of the system.

The procedure-based adaptation facilitates ease-of-use of the equipment and improves staff comfort also in an ergonomic sense by the adaptation of the lab, targeted equipment such as a user interface, to a specific user or task. Being based on image detection, the adaption can be free of manual interaction. As an option, user correction is provided by allowing overriding, e.g. by manual correction or manipulation of the adapted control signal.

The procedure-based adaptation of the medical equipment supports in avoiding, or at least minimizing, any distraction physical or mental stress to the clinical staff caused by the sometimes abundant use of electronic equipment to support in focusing on medical treatment and patient care as the ultimate concern.

The procedure-based adaptation can also be linked to an automatic position control functionality on the tableside module of an X-ray system in order to recall C-arc positions as an application for the determined adjustment values. Such procedure-based adaptation could also be linked to a recall functionality of the subject support, like a patient table.

In an example, the image detection is based on image data from cameras that are used to observe the cath lab situation, for example for recording purposes regarding interventional procedures. Such cameras can be arranged for example on a monitor rack suspension or mounted by ceiling suspension. In an example, camera spatial registration is provided, for example by spatial registration of the monitor rack camera or the ceiling camera. When the camera is mounted to a movable support, continuous spatial registration may be provided. However, it is also provided to spatially register the image provided by the camera by detecting designated markers like landmarks in the image.

The procedure-based adaptation results for example in a smart cath lab system able to perform role and/or activity detection in video images acquired with cameras attached to or in the neighborhood of Cath lab equipment. The cameras register the individual medical staff members, the patient and or the status of certain system components in the room, e.g. the C-arc or table position. It is possible to adapt the various environmental or user interface conditions in an intervention room, based on the detected role of a staff member, or a specific activity. The medical staff perceives and experiences the specific environmental changes as an adaptive intelligent behavior of the cath lab itself, e.g. including adapted lighting conditions in the room based on the detected phase of the intervention, e.g. start of an X-ray procedure (for example, with dimmed lights) or start of the cleaning of the room (for example, with bright lights).

Another example is to provide settings of an X-ray procedure type based on the procedure type, recognized by the system, or the role of the user. For instance, the room may be used for percutaneous coronary interventional procedures and the procedure-based adaptation adapts the equipment to corresponding operation settings upon detecting respective staff or activity in the images, for example preparation activity. A further example is the adapted appearance of a user interface e.g. at the tableside or other control units based on the face of the user and the detected role, recognized by the system. A still further example is an adaptation of table or C-arc position based on the phase of the intervention, e.g. automatic parking of the C-arc at the end of the patient treatment.

As a result, a camera-based grabbing and image evaluation system is provided that results in an occurrence of various specific smart actions and changes in the cath lab environment or the relevant user interface adaptations, based on the camera observations. Simply said, actions are caused by the detected activity or role.

The procedure-based adaptation can be provided e.g. within X-ray cath labs with different kind of X-ray equipment during a minimal invasive intervention. The procedure-based adaptation is suitable to cath labs equipped with cameras to detect various objects and situations.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for procedure-based adaptation of medical equipment.
Fig. 2 shows a perspective view of an example of a system for procedure-based adaptation of medical equipment in an interventional space.
Fig. 3a shows a rack-mounted camera as an example for the image capturing arrangement.
Fig. 3b shows a ceiling-mounted camera as another example for the image capturing arrangement.
Fig. 4 shows an image as captured by the camera of Fig. 3b.
Fig. 5 shows an adapted user interface as an example for an adapted medical equipment.
Fig. 6 shows basic steps of an example of a method for procedure-based adaptation of medical equipment.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

The term "subject" may also be referred to as individual. The "subject" may further also be referred to as patient, although it is noted that this term does not indicate whether any illness or disease is actually present with the subject.

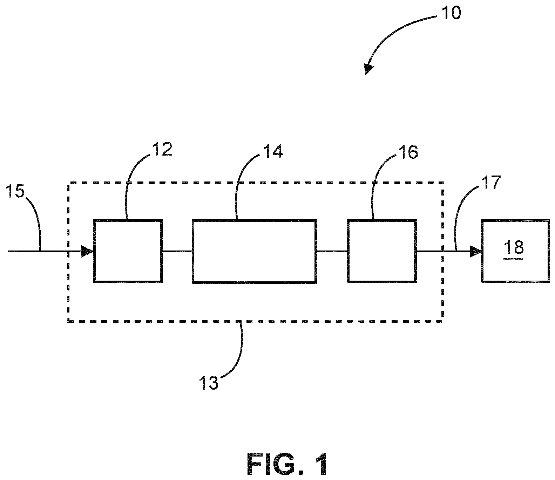

Fig. 1 schematically shows an example of a device 10 for procedure-based adaptation of medical equipment. The device 10 comprises an image data receiver 12, a processor 14 and an equipment adaptor 16. The image data receiver 12 is configured to receive image data of a situation in a medical procedure zone comprising medical equipment, which image data shows at least a part of an activity in the medical procedure zone. The processor 14 is configured to detect at least one of the group of individuals and medical equipment in the medical procedure zone and their corresponding activities based on the image data, and to recognize activity patterns and roles based on the detected activities. The processor 14 is also configured to analyze adaptation possibilities of the medical equipment based on the recognized activity patterns and roles, and to determine adjustment values for the medical equipment based on the analyzed adaptation possibilities. The equipment adaptor 16 is configured to provide adapted control signals 18 for the medical equipment based on the determined adjustment values for a procedure-based adaptation of the medical equipment.

A dotted frame 13 indicates that the image data receiver 12, the processor 14 and the equipment adaptor 16 can be provided in an integrated or combined manner. However, the image data receiver 12, the processor 14 and the equipment adaptor 16 can also be provided separately in a data-connected manner. A first arrow 15 indicates the input of the image data, and a second arrow 17 indicates the output of the adapted control signals 18.

The term "medical procedure zone" relates to a room or space with medical equipment, such as imaging equipment, e.g. diagnostic imaging equipment. The medical procedure zone is a designated zone for a medical procedure like a medical examination, a medical diagnosis, a medical treatment, a medical intervention and a medical operation or the like, which procedure requires the presence of a particular technical environment, for example requiring specific lighting, imaging, monitoring or supporting appliances.

The medical procedure zone is also referred to as medical examination zone, medical intervention zone or medical operation zone. The medical procedure zone is also referred to as medical procedure room or medical procedure space. For example, the term examination room or operation room is used. In an example, the medical procedure zone is a catherization laboratory, or cath lab, i.e. an examination zone, area or room for examination-or intervention-procedures, in which catheters are used.

The term "individuals" in "individuals and medical equipment" relates to individuals that are present in the medical procedure zone. The term individual relates to, for example, a subject or individual of interest, e.g. a patient, but also to medical personnel such as doctors, surgeons, nurses or radiologists and other staff members that are present during a medical procedure.

The term "medical equipment" in "individuals and medical equipment" relates to appliances and devices used in the examination zone. For example, medical equipment comprises subject supports, medical imaging devices, display devices, lighting arrangements, operation supply units, supplies of interventional devices and utilities, or of auxiliary material support material and media supply like oxygen supply, energy supply, air supply, extraction of air and other disposables, or data supply. Medical equipment relates to components, devices or systems specifically designed for medical purposes, but also to other components, devices or systems that are not specifically designed for such medical purposes, but which are intended to be used for medical purposes during a medical examination. Medical equipment relates to equipment for use in medical procedures such as examinations or interventions of subjects. As an example, medical equipment relates to imaging equipment, monitoring equipment, subject supporting equipment, diagnostic equipment or treatment equipment. The term "medical equipment" also relates to equipment used in an operational zone, such as interventional tools, surgical tools, catheters, guidewires, operational support materials and devices, prothesis or auxiliary devices like sponges.

The term "situation" relates to the scenario in the medical procedure zone as shown in the image(s). If the images are live images, the situation is the actual or current situation. The situation may also be referred to as scenario.

The term "activities" relates to actions or motions performed, i.e. done, by the individuals in the medical procedure zone. The term "activities" also relates to movements or operations performed, i.e. executed, by equipment in the medical procedure zone.

The term "activity patterns" relates to an action or motion with a characterizing course of the motion or movement. Activity patterns may be provided as predefined patterns by identifying certain basic pattern or may be provided in form of a self-learning process. The patterns may be defined by certain steps in a certain order or may also be defined by certain trajectories of certain parts, devices and equipment or of body parts of an individual or a combined action by two or more individuals.

The term "roles" relates to an action or motion with a certain function or task within the medial examination space, for example to monitor the subject or to anaesthetize a subject in a controlled manner. An example for a role is a radiologist technician responsible for aligning an X-ray imaging system and for activating same. Another example for a role is a nurse or assistant supplying sterilized devices to a user like a surgeon. A still further example of a role is a patient taking up a defined position or performing a gesture.

The term "image data receiver" relates to a device that serves as an incoming interface or input for the processor. The image data receiver can be provided as a separate device or unit or can be provided integrated with the processor or the equipment adaptor.

The term "processor" relates to a device that serves for processing, i.e. computing data such as image processing. The processor is thus a data processing arrangement, for example a computer. The processor can be provided as a separate device or unit or can be provided integrated with the image data receiver or the equipment adaptor.

The term "equipment adaptor" relates to a device that serves as an outgoing interface or output for adapting the medical equipment. The equipment adaptor thus provides control signals or other steering commands or instructions for the medical equipment to arrange for the adaptation of the medical equipment. The equipment adaptor can be provided as a separate device or unit or can be provided integrated with the processor or the image data receiver.

The term "to detect" relates to image content analysis based on the image data in order to find and identify individuals and medical equipment. The detection is provided purely image based and may be conducted for a particular image or may be conducted for a sequence of images.

The term "to recognize" relates to e.g. comparing detected movements, motions or gestures with predefined or trained activity patterns. Based on a predefined matching degree, the detected movements, motions or gestures are assigned to the activity patterns.

The term "to analyze" relates to determine possible activity deviations or changes and to assess and evaluate suitability or benefits for such activity deviations or changes.

The term "to determine" relates to identifying an activity deviation or change based on the assessment and evaluation and to calculate adjustment values for achieving the activity deviation or change.

The term "adapted control signals" relates e.g. to signals that control the operation and functionality of the medical equipment. For example, the adapted control signals may be provided as a correction signal for an existing control signal. As another example, the adapted control signals may be provided as a corrected control signal replacing an existing control signal.

The term "procedure-based adaptation" relates to modifying medical equipment depending on an actual situation or situation.

The images may be provided as a sequence of images of the situation. The images may also be provided as multiple sequences of images of the situation taken from different point of views.

In an example, the processor detects and analyses roles in the activities, compares these roles to predetermined roles of an interventional procedure, and determines the adjustment values for the medical equipment based on values assigned to the predetermined roles.

The adapted control signals 18 comprise technically related data resulting in an adaptation of the medical equipment.

In another example, the image data receiver 12 is configured to receive a plurality of video images as the image data, the video images capturing at least one of the group of: staff members, subject of interest and appliances and components in the medical procedure zone.

In an option, the processor 14 is configured to detect and analyze roles in the activities, to compare these roles to predetermined roles of an interventional procedure, and to determine the adjustment values for the medical equipment based on values assigned to the predetermined roles.

In a further option, the processor 14 is configured to predict activities and roles to follow the detected activities based on the recognized activity patterns and roles, and to analyze adaptation possibilities of the medical equipment based on the predicted activity patterns and roles, and to determine the adjustment values 18 based on the analyzed adaptation possibilities.

In an example, the processor 14 is provided with an artificial intelligence deep learning device for role or activity detection. Alternatively, or in addition, the processor 14 is provided with an artificial neural network comprising a model embedded in the processor for processing the image data.

The image content processing is based on identifying structures within the image and assigning these to stored patterns for identifying for example a staff member. The image content processing is based on available image recognition techniques. However, the recognition to activity patterns and roles, as well as the analysis of adaptation possibilities is based on a predefined assignment to predetermined patterns and roles. For this purpose, a plurality of standard patterns is defined as a basis and deviations and variations are further identified by self-learning procedures. Thus, an increasing database is created und constantly updated.

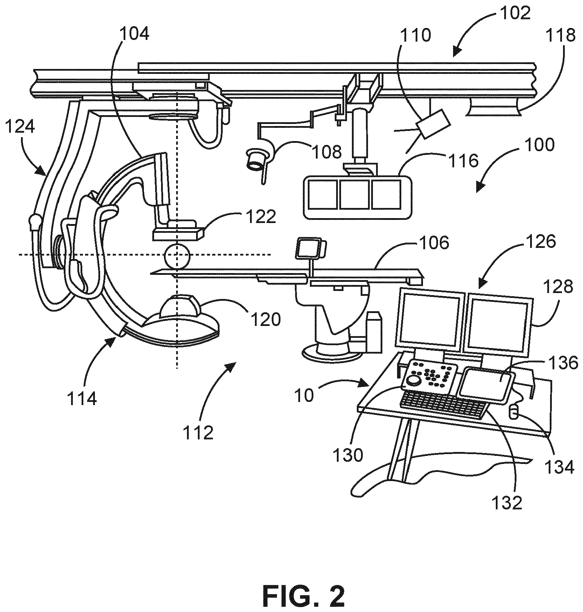

Fig. 2 shows a perspective view of an example of a system 100 for procedure-based adaptation of medical equipment in an interventional space. The system 100 comprises an image capturing arrangement 102. The system 100 also comprises one of the examples of the devices 10 for procedure-based adaptation of medical equipment described above and below. The image capturing arrangement 102 provides a plurality of images as the image data to the image data receiver 12 (not shown in detail). The equipment adaptor 16 (also not shown in detail) is configured to provide the adapted control signals 18 for at least a part of the medical equipment based on the image data from the image capturing arrangement 102.

As an option, a C-arm 104 for X-ray imaging or a subject support table 106 is provided. The components may also comprise movable lighting 108.

Fig. 2 indicates, as an option, that the system 100 may be provided as a cathlab system comprising medical equipment 112. The equipment adaptor provides the adapted control signals to at least a part of the medical equipment 112, and at least a part of the medical equipment 112 operates according the adapted control signals.

As also indicted in Fig. 2 as an option, the medical equipment 112 comprises at least one of the group of medical imaging device 114, for example an X-ray imaging device, one or more displays 116 and lighting arrangement 118.

Fig. 2 shows the X-ray imaging device with an X-ray source 120 and an X-ray detector 122 movably mounted to the C-arm 104. An object 124 can be arranged on the subject support table 106 between the X-ray source 120 and the X-ray detector 122. The C-arm 104 may be mounted to the ceiling with a mounting structure 124.

The device 10 for procedure-based adaptation of medical equipment may be provided in the context of a control 126 shown in the foreground with displays 128 and control interfaces 130 like a keyboard 132, a mouse 134 or a graphic tablet 136.

As another example, the medical imaging device 114 is an MRI device, a CT-system or an ultrasound device (not shown).

In an example, the display 116 comprises at least one of the group of a main monitor, an auxiliary monitor, a beamer, a head mounted display and a projector.

In an example, the lighting arrangement 118 is provided as a room lighting arrangement. The lighting arrangement 118 may comprise fixedly installed lights, portable lights and/or movable lights. The lighting arrangement may also comprise room lighting or environment lighting. The lighting arrangement may further comprise lighting for illuminating one or more areas of interest. The lighting arrangement may also relate to lighting attached to operational or imaging equipment. In an example, the room lighting is adapted based on the detected activities. For example, if an X-ray imaging procedure, i.e. a radiation procedure, is detected based on the images, room lighting may be dimmed. If a room cleaning procedure is detected, the room lighting may be changed to bright lights.

The medical equipment 112 may also comprise at least one of the group of environmental conditioning arrangement and operation supply units (not shown in detail). In an example, the environmental conditioning arrangement comprises at least one of the group of air ventilation, air supply and air conditioning. For example, the environmental conditioning arrangement comprises at least one of the group of air ventilation, i.e. air fan for air supply and air conduits for air extraction, air temperature adjustment, i.e. heater and/or cooler, and air humidity adjustment, i.e. air humidifier or air dryer. In an example, the term "operation supply units" relates to oxygen supply, other gaseous medium supply, liquid supply like fresh water supply, or energy supply for an operation.

In an example, the medical equipment 112 comprises a control for environmental conditions in the medical procedure zone comprising at least one of the group of air, light, temperature and acoustics. The control for environmental conditions is adapted based on the detected activities such that the environmental conditions are adjusted to the situation.

Another example for the medical equipment 112 is a subject support. The term "subject support" relates to e.g. the subject table 106, like a patient table, or another type of subject support, for example for chest X-ray imaging or mammography.

Further examples (not shown in Fig. 2) for the medical equipment 112 are supply of interventional devices and utilities, and auxiliary material support. At least one of the above group operates according the adapted control signals 18. In an example, the term supply of interventional devices and utilities relates to providing tools and parts to the staff members, for example arranged in containers, or on trays or shelfs.

In an example, the term "auxiliary material support" relates to providing material like clamps, sponges, absorbing tissue, disinfection, bandages, adhesive tapes or swabs.

In an example, the medical equipment comprises a control for environmental conditions of the interventional space, and the environmental conditions are adapted based on the detected activities.

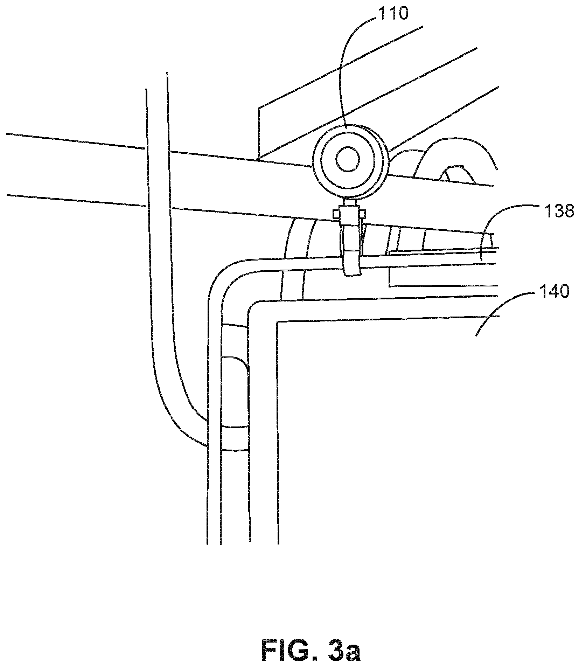

Fig. 3a shows a camera 110 as an example for the image capturing arrangement 102. The camera 110 is an optical camera providing live images as the plurality of images to provide the adapted control signals based on the current situation. For example, the camera 110 is mounted to a support structure 138 of a fixedly installed display 140. The display can also be movable, if spatial registration of the camera 110 is provided.

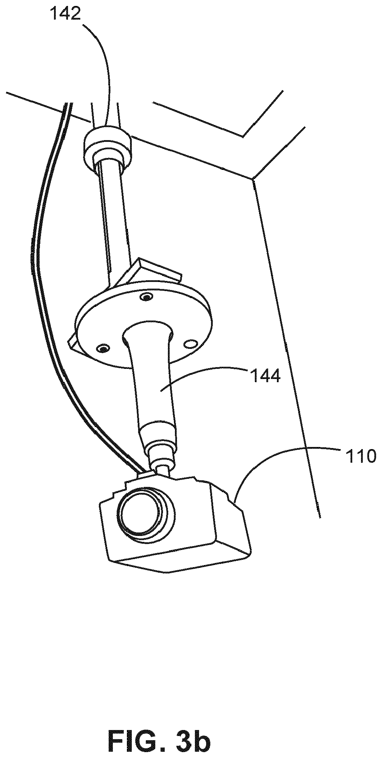

Fig. 3b shows a further example of the camera 110 as another example for the image capturing arrangement. The camera is attached to a building structure 142 by a mounting structure 144.

The at least one optical camera operates under visible light conditions. In an option, the at least one optical camera operates outside visible light conditions with infrared or ultraviolet light. In another option, at least one optical camera is provided that operates under a combination of visible and invisible light condition.

In an option, a plurality of cameras is spatially registered within a reference system of an operational room (also referred to as operational theatre). The video images are registered to the reference system.

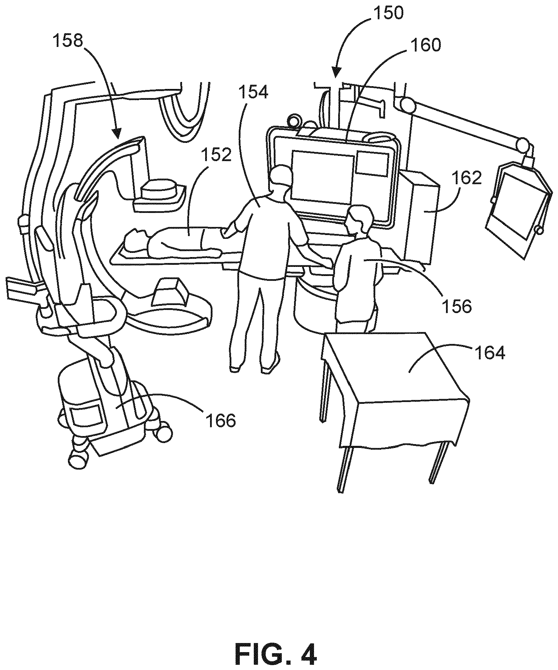

Fig. 4 shows an image 150 with a situation as captured by the camera of Fig. 3b. A patient 152 is arranged on a support; a first staff member 154, e.g. a doctor, is standing next to the support, and a second staff member 156, e.g. a nurse, is standing next to the first staff member 154. Further, a C-arm X-ray imaging system 158 is provided to the left (in the image) of the support, and a monitor arrangement 160 to the right (in the image). Further medical equipment is provided like a control apparatus 162, for example for environmental conditions, and a table 164 covered by a sterile cloth. A further intervention or examination apparatus 166 is shown in the left foreground in the image. Based on the image data, the situation can be analyzed in so far as activity patterns and roles can be identified to provide a respective adapted control of the medical equipment.

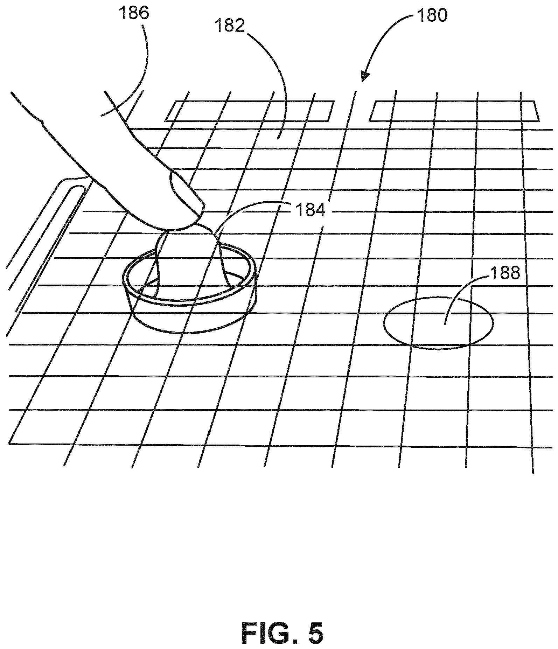

Fig. 5 shows an adapted user interface 180 as an example for an adapted medical equipment. The user interface 180 is adapted based on the detected activities. As shown in Fig. 5 as an example, the user interface 180 is a graphical user interface 182 and the graphical appearance is adapted based on the detected activities. For example, based on a detected activity, unused user interface elements are temporarily hidden, i.e. de-activated and not shown. Only a currently used control knob 184 is shown on the graphical user interface 182. A finger 186 indicates the interactive operation of the displayed (virtual) control knob 184. A further spot 188 indicates a hidden control element.

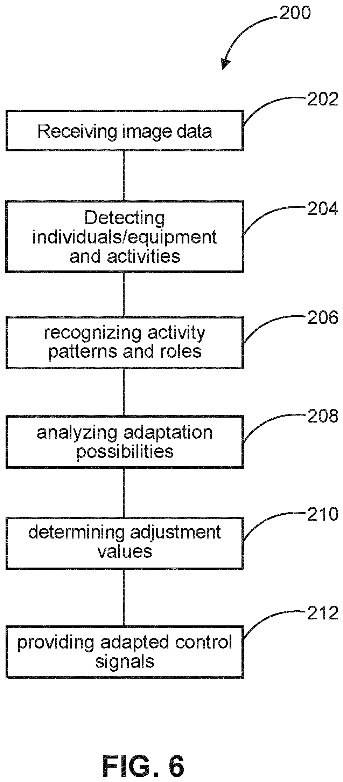

Fig. 6 shows an example of basic steps of a method 200 for procedure-based adaptation of medical equipment. The method 200 comprises the following steps:
- In a first step 202, image data of a situation in a medical procedure zone comprising medical equipment is received, which image data shows at least a part of an activity in the medical procedure zone.
- In a second step 204, at least one of the group of individuals and medical equipment in the medical procedure zone and their corresponding activities are detected based on the image data.
- In a third step 206, activity patterns and roles are recognized based on the detected activities.
- In a fourth step 208, adaptation possibilities of the medical equipment are analyzed based on the recognized activity patterns and roles.
- In a fifth step 210, adjustment values for the medical equipment are determined based on the analyzed adaptation possibilities.
- In a sixth step 212, adapted control signals for the medical equipment are provided based on the determined adjustment values for a procedure-based adaptation of the medical equipment.

As a further option, not further indicated, it is provided a seventh step of operating at least a part of the medical equipment according to the adapted control signals.

In an example, the method steps are provided in the above-mentioned order. In an option, the detecting step (i.e. second step 204) and the recognizing step (i.e. third step 206) are provided as a combined step. In another option, the recognizing step 206 and the analyzing step (i.e. fourth step 208) are provided as a combined step. In still another option, the analyzing step 208 and the determining step (i.e. fifth step 210) are provided as a combined step. In a further option, the detecting step 204, the recognizing step 206 and the analyzing step 208 are provided as a combined step. In a still further option, the recognizing step 206, the analyzing step 208 and the determining step 210 are provided as a combined step. In a further option, the detecting step 204, the recognizing step 206, the analyzing 208 step and the determining step 210 are provided as a combined step.

In an example, a plurality of video images is received as the image data, wherein the video images are capturing at least one of the group of: staff members, subject of interest and appliances and components in the medical procedure zone.

In an option, it is provided to detect and analyze roles in the activities, to compare these roles to predetermined roles of an interventional procedure, and to determine the adjustment values for the medical equipment based on values assigned to the predetermined roles.

In an example, following the recognizing of the activity patterns and roles, a step is provided of predicting activities and roles to follow the detected activities based on the recognized activity patterns and roles, and, as the analyzing step, a step of analyzing adaptation possibilities of the medical equipment based on the predicted activity patterns and roles, and, as the determining step, a step of determining the adjustment values based on the analyzed adaptation possibilities.

In another option, for the step of role or activity detecting, an artificial intelligence deep learning is provided.

In a further option, at least one of the steps of detecting, recognizing, analyzing and determining are provided by using an artificial neural network comprising a model embedded in the processor for processing the image data.

In order to provide further relief for staff members, a device for procedure-based adaptation of medical equipment comprises an image data receiver as an input, a processor and an equipment adaptor as an output. Images of a situation that show activity in a medical procedure zone are used to detect individuals and medical equipment in the medical procedure zone and their corresponding activities, and to recognize activity patterns and roles based on the detected activities. Adaptation possibilities of the medical equipment are analyzed and adjustment values for the medical equipment is determined. The equipment adaptor provides adapted control signals for the medical equipment based on the determined adjustment values. In an example, in a cath lab, a system comprises an image capturing arrangement with cameras that provide the images. The adapted control signals are used for adapting medical equipment of the cath lab.

In an example, a computer program element is provided enabling a processor to carry out the method of one of the examples described above.

In an example, a computer program or program element for controlling an apparatus according to one of the examples above is provided, which program or program element, when being executed by a processing unit, is adapted to perform the method steps of one of the method examples above.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

In an example, a computer readable medium is provided having stored the above program element.
Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs). This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for procedure-based adaptation of medical equipment, the device comprising:
- an image data receiver (12);
- a processor (14); and
- an equipment adaptor (16);
- wherein the image data receiver (12) is configured to receive image data of a situation in a medical procedure zone comprising medical equipment, which image data shows at least a part of an activity in the medical procedure zone;
- wherein the processor (14) is configured to detect at least one of the group of individuals and medical equipment in the medical procedure zone and their corresponding activities based on the image data, to recognize activity patterns and roles based on the detected activities, to analyze adaptation possibilities of the medical equipment based on the recognized activity patterns and roles, and to determine adjustment values for the medical equipment based on the analyzed adaptation possibilities; and
- wherein, for a procedure-based adaptation of the medical equipment, the equipment adaptor (16) is configured to provide adapted control signals (18) for the medical equipment based on the determined adjustment values.

2. Device according to claim 1, wherein the adapted control signals (18) comprise technically related data resulting in an adaptation of the medical equipment.

3. Device according to claim 1 or 2, wherein the image data receiver (12) is configured to receive a plurality of video images as the image data, the video images capturing at least one of the group of: staff members, subject of interest and appliances and components in the medical procedure zone.

4. Device according to claim 1, 2 or 3, wherein the processor (14) is configured to detect and analyze roles in the activities, to compare these roles to predetermined roles of an interventional procedure, and to determine the adjustment values for the medical equipment based on values assigned to the predetermined roles.

5. Device according to one of the claims 1 to 4, wherein the processor (14) is configured to predict activities and roles to follow the detected activities based on the recognized activity patterns and roles, and to analyze adaptation possibilities of the medical equipment based on the predicted activity patterns and roles, and to determine the adjustment values based on the analyzed adaptation possibilities.

6. Device according to one of the preceding claims, wherein the processor (14) is provided with:
- an artificial intelligence deep learning device for role or activity detection; and/or
- an artificial neural network comprising a model embedded in the processor for processing the image data.

7. A system (100) for procedure-based adaptation of medical equipment in an interventional space, the system comprising:
- an image capturing arrangement (102); and
- a device (10) for procedure-based adaptation of medical equipment (112) according to one of the preceding claims;
wherein the image capturing arrangement (102) provides a plurality of images as the image data to the image data receiver (12); and
wherein the equipment adaptor (16) is configured to provide the adapted control signals (18) for at least a part of the medical equipment (112) based on the image data from the image capturing arrangement (102).

8. System according to claim 7, wherein the image capturing arrangement (102) comprises at least one optical camera (110) providing live images as the plurality of images to provide the adapted control signals (18) based on the current situation.

9. System according to claim 7 or 8, wherein the system is a cathlab system comprising the medical equipment (112);
wherein the equipment adaptor (16) provides the adapted control signals (18) to at least a part of the medical equipment (112); and
wherein at least a part of the medical equipment (112) operates according the adapted control signals (18).

10. System according to one of the claims 7 to 9, wherein the medical equipment (112) comprises at least one of the group of:
- medical imaging device,
- display,
- lighting arrangement,
- environmental conditioning arrangement,
- operation supply units,
- subject support,
- supply of interventional devices and utilities, and
- auxiliary material support; and
- wherein at least one of the above group operates according the adapted control signals (18).

11. System according to one of the claims 7 to 10, wherein the medical equipment (112) comprises a user interface (180); and
wherein the user interface (180) is adapted based on the detected activities.

12. System according to one of the claims 7 to 11, wherein the medical equipment (112) comprises a control for environmental conditions in the medical procedure zone comprising at least one of the group of air, light, temperature, acoustics and; and
wherein the control for environmental conditions is adapted based on the detected activities such that the environmental conditions are adjusted to the situation.

13. A method (200) for procedure-based adaptation of medical equipment, comprising the following steps:
- receiving (202) image data of a situation in a medical procedure zone comprising medical equipment, which image data shows at least a part of an activity in the medical procedure zone;
- detecting (204) at least one of the group of individuals and medical equipment in the medical procedure zone and their corresponding activities based on the image data;
- recognizing (206) activity patterns and roles based on the detected activities;
- analyzing (208) adaptation possibilities of the medical equipment based on the recognized activity patterns and roles;
- determining (210) adjustment values for the medical equipment based on the analyzed adaptation possibilities; and
- providing (212) adapted control signals for the medical equipment based on the determined adjustment values for a procedure-based adaptation of the medical equipment.

14. A computer program element enabling a processor to carry out the method of claim 13.

15. A computer readable medium having stored the program element of claim 14.
